# EUROPÄISCHE PATENTANMELDUNG

(11) **EP 1 925 610 A1**
(43) Veröffentlichungstag der Anmeldung: **28.05.2008**
(21) Anmeldenummer: 06024436.5
(22) Anmeldetag: 24.11.2006
(51) Int. Cl.: C07C 239/20, C07C 249/12, C07C 251/54

(54) **Verfahren zur Herstellung von 2-Aminooxyethanol**

(71) Anmelder: Bayer CropScience AG, 40789 Monheim (DE)
(72) Erfinder: Pazenok, Sergiy, 42699 Solingen (DE); Lui, Norbert, 51519 Odenthal (DE)

(57) **Zusammenfassung**

Die Erfindung betrifft ein Verfahren zum Herstellen von Aminoglykol 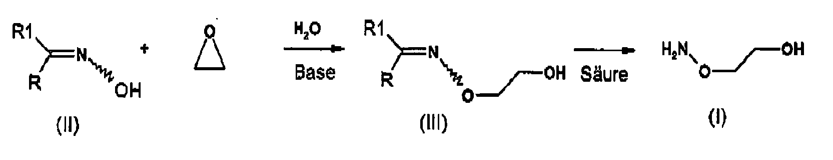 durch Umsetzung von Ketoximen mit Ethylenoxid unter basischen Bedingungen zu einem substituierten 2-Hydroxyethylketoxim und dessen anschließende Umsetzung mit einer Säure zu Aminoglykol.

## Beschreibung

Die Erfindung betrifft ein Verfahren zum Herstellen von Aminoglykol durch Umsetzung von Ketoximen mit Ethylenoxid unter basischen Bedingungen zu einem substituierten 2-Hydroxyethylketoxim und dessen anschließende Umsetzung mit einer Säure zu Aminoglykol.

Aminoglykol ist ein wichtiger Synthesebaustein zur Herstellung von herbizid wirksamen Agrowirkstoffen, insbesondere zur Herstellung von Dioxazinderivaten, insbesondere von Dioxazin-Pyridinyl-Suflonylharnstoffen (US 5,476,936)

So lehrt EP0655437 ein Verfahren zum Herstellen von Aminoglykol durch Umsetzung von Acetonoxim mit Ethylencarbonat in Gegenwart von DBU, wobei jedoch lediglich Umsatzausbeuten von 65-75% erzielt werden. Zusätzlich handelt es sich bei DBU um einen teuren Einsatzstoff, welcher nicht zurückgewonnen werden kann.

In der US-A-4,687,849 wird ein Verfahren zur Herstellung von 2-(Isopropylidenamino)oxyethanol vorgeschlagen, bei dem Acetonoxim in Wasser mit Ethylenoxid umgesetzt wird. Eine Reaktionsausbeute wurde nicht angegeben. Das Produkt 2-Hydroxyethylacetonoxim ist sehr gut wasserlöslich und schwierig durch Extraktion zu isolieren. Durch Nacharbeitung des in der US-A-4,687849 (Beispiel 1) angegebenen Reaktionsweges ergibt sich aber, dass die Ausbeute dieser Reaktion 50-55 % nicht überschreitet, da als Nebenreaktion die Alkylierung von Stickstoff und die Bildung von Nitron stattfindet. Nitrone sind höchst unerwünschte Nebenprodukte da Sie sich bereits beim Aufarbeiten zersetzen, und lhre Isolierung wegen des hohen Zersetzungspotenzials unerwünscht ist.

Eine weitere Methode zur Herstellung vom Aminoglykol (J.Chem.Soc. Chem.Com., 1986, 903) geht von 2-Bromethanol und N-Hydroxyphthalimid und anschließender Spaltung mit Hydrazinhydrat aus, was jedoch sehr hohe Herstellkosten mit sich bringt.

Somit kann gesagt werden, dass die im Stand der Technik beschriebenen Verfahren den Nachteil aufweisen, dass sie (a) entweder sehr teuer sind und/oder (b) die Ausbeuten nicht hoch genug sind, um eine technische Realisierung dieses Reaktionsschritts zu betreiben und/oder dass (c) das gewünschte Aminoglykol nicht einfach und kostengünstig von den Nebenreaktionsprodukten abgetrennt und isoliert werden kann.

Der vorliegenden Erfindung liegt daher die Aufgabe zugrunde, ein einfacheres und wirtschaftlicheres Verfahren zur Herstellung von Aminoglykol zur Verfügung zu stellen.

Die zuvor beschriebene Aufgabe wurde erfindungsgemäß gelöst durch ein Verfahren zum Herstellen von Aminoglykol der Formel (I) 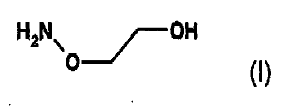 als Schritte enthaltend
(a) die Umsetzung von Ketoximen der allgemeinen Formel (II) mit Ethylenoxid in wässriger Lösung und in Gegenwart einer Base 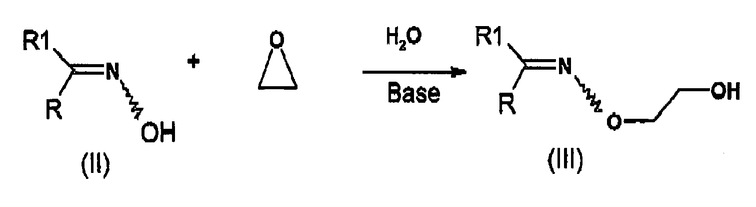 wobei
   - R1 und R: unabhängig voneinander unsubstituiertes oder durch Halogen, CN, NO₂, einfach oder mehrfach substituiertes lineares (C₁-C₈)-Alkyl, Phenyl oder verzweigtes (C₃-C₈)-Alkyl bedeuten, aber für
   den Fall, dass R1 oder R ein unsubstituiertes oder durch Halogen, CN, NO₂ substituiertes (C₁-C₈)-Alkyl ist, der jeweils andere Rest R1 oder R einem unsubstituierten oder durch Halogen, CN, NO₂ substituierten Phenyl oder einem verzweigten (C₃-C₈)-Alkyl entspricht
   und (b) 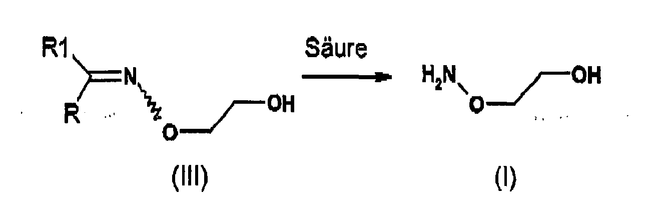 die Weiterreaktion des gemäß des Reaktionsschritts (a) entstandenen Iminooxyethanols der allgemeinen Formel (III) in Gegenwart einer Säure zum Aminoglykol.

Bevorzugt haben in Verbindungen der Formeln (II) und (III) R1 und R die Bedeutung von Methyl, Ethyl, Propyl, Phenyl, sek.-Butyl, tert.-Butyl, isobutyl, lsopropyl oder Neopentyl, wobei die vorgenannten Reste jeweils unabhängig voneinander unsubstituiert oder durch Halogen, CN, NO₂, einfach oder mehrfach substituiert sind, aber für den Fall, dass R1 oder R ein unsubstituiertes oder durch Halogen, CN, NO₂ substituiertes Methyl, Ethyl oder Propyl ist, entspricht der jeweils andere Rest R1 oder R einem unsubstituierten oder durch Halogen, CN, NO₂ substituierten Phenyl, sek.-Butyl, tert-Butyl, Isobutyl, Isopropyl oder Neopentyl.

Weiter bevorzugt haben in Verbindungen der Formeln (II) und (III) R1 und R die Bedeutung von Methyl, Ethyl, Phenyl, sek.-Butyl, tert.-Butyl, Isobutyl, Isopropyl und

Neopentyl wobei für den Fall, dass R1 oder R ein Methyl oder Ethyl ist, der jeweils andere Rest R1 oder R ausgewählt sein muss aus der Gruppe Phenyl, sek.-Butyl, tert.-Butyl, Isobutyl oder Isopropyl.

Besonders bevorzugt entspricht in Verbindungen der Formeln (II) und (III) R1 einem Methyl und R einem tert-Butyl, oder Isobutyl, oder aber R entspricht einem Methyl und R1 einem tert-Butyl, oder Isobutyl.

Ganz besonders bevorzugt entspricht in Verbindungen der Formeln (II) und (III) R1 einem Methyl und R einem tert.-Butyl, oder aber R entspricht einem Methyl und R1 einem tert.-Butyl.

Weitere Ausführungsformen der vorliegenden Erfindung können den abhängigen Ansprüchen und der Beschreibung entnommen werden.

Das erfindungsgemäße Verfahren kann anhand des folgenden Schemas (1) erläutert werden:

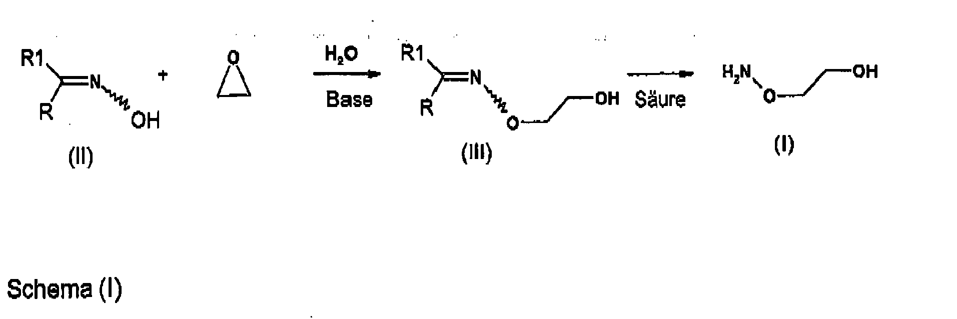

Ein weiterer Vorteil des erfindungsgemäßen Verfahrens besteht (wie im nachstehenden Schema (II) dargestellt) darin, dass das Produkt der Formel (III) in Wasser nicht löslich ist und somit ohne, gesonderte Extraktionsschritte aus der wässrigen Phase isoliert werden. Durch das Verseifen des 2-Hydroxyethylketaxims der Formel (III) kann das Aminoglkykol quantitativ freigesetzt werden. Dabei ist das gebildete Keton (wie bespielsweise das Methyl-tert.-butylketon für R1=Methyl und R=tert,-Butyl (Pinakolon)) nicht in Wasser löslich und kann daher vollständig, wie beispielsweise durch Zugabe von Hydroxylamin, zurückgeführt und anschließend wieder für die Herstellung von Ketoximen verwendet werden.

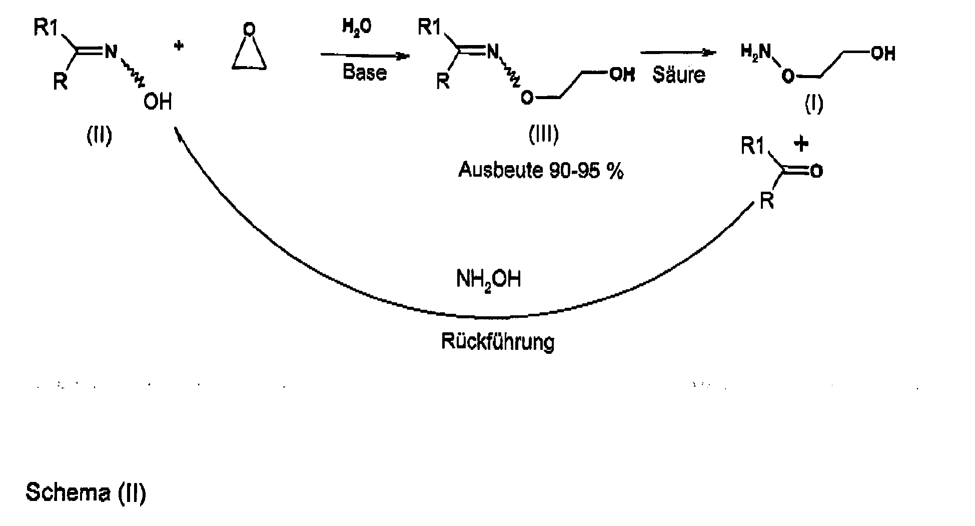

Insgesamt ist das neue Verfahren somit den aus dem Stand der Technik bekannten Verfahren sowohl aus ökonomischer (geringe Kosten der Ausgangsmaterialien und der Hilfsstoffe, hohe Produktausbeute sowie Rückgewinnung des Ketons (nach Schema (II))), als auch aus ökologischer Sicht, d.h der Entstehung geringerer

Abfallmengen, deutlich überlegen.

Im Zusammenhang mit der vorliegenden Erfindung umfasst der Begriff Halogene, solche Elemente, die ausgewählt sind aus der Gruppe bestehend aus Fluor, Chlor, Brom und lod, wobei Fluor, Chlor und Brom bevorzugt und Fluor und Chlor besonders bevorzugt verwendet werden.

Substituierte Reste können einfach oder mehrfach substituiert sein, wobei bei Mehrfachsubstitutionen die Substituenten gleich oder verschieden sein können.

Als Basen können sowohl organische wie auch anorganische Basen verwendet werden. Bevorzugt werden anorganische Basen wie beispielsweise LiOH, NaOH, KOH, Ca(OH)₂, Ba(OH)₂, Li₂CO₃, K₂CO₃, Na₂CO₃, NaHCO₃, oder organische Basen wie Amine (wie beispielsweise bevorzugt Triethylamin, Diethyllsopropylarnin), Bu₄NOH, Piperidin, Morpholin, Alkylpyridine, verwendet. Besonders bevorzugt werden anorganische Basen, ganz besonders bevorzugt, LiOH, NaOH und KOH verwendet.

Als Säuren können sowohl organische wie auch anorganische Säuren verwendet werden. Bevorzugt werden anorganische Säuren wie beispielsweise HCI, HBr, HF, H₂SO₄, H₃PO₄ oder organische Säuren wie CF₃COOH, CH₃COOH, p-Toluolsulfonsäure verwendet. Besonders bevorzugt worden anorganische Säuren, ganz besonders bevorzugt HCI und H₂SO₄ verwendet.

Das Verfahren kann sowohl in Wasser als auch in Gegenwart eines inerten organischen Lösungsmittels, bevorzugt eines polaren aprotischen Lösungsmittels durchgeführt werden. Beispiele von organischen Lösungsmitteln sind aromatische oder aliphatische Lösungsmittel wie Benzol, Toluol, Xylol, Mesytilen, Hexan, Heptan, Octan, Cyclohexan, aliphatische und aromatische Halogenwasserstoffe wie Methylenchlorid, Dichlorethan, Chloroform, Kohlenstofftetrachlorid, Chlorbenzol, Dichlorbenzol, Ether wie Diethylether, Dibutylether, Diisobutylether, Methyl-tert-butylether, Isopropylethylether, Tetrahydrofuran, und Dioxan; weiter auch Dimethylsulfoxid, und Säureamidderivate, wie N,N-dimethylformamid, N,N-Dimethylacetamid und N-Methyl-2-pyrollidon, sowie auch Carbonsäureester wie Ethylacetat, oder aber auch Dioxan, Diglym, Dimethylglycol oder THF; Nitrile wie Methylnitril, Butylnitril oder Phenylnitril. Besonders bevorzugt sind Toluol, Xylol, Dichlorbenzol, Chlorbenzol oder Ethylacetat.

Die Verbindungen der Formeln IIl und III können als Mischungen verschiedener möglicher isomerer Formen, insbesondere von Stereoisomeren, wie z.B. E- und Z-, syn und anti, sowie optischen lsomeren, gegebenenfalls aber auch von Tautomeren vorliegen. Es werden sowohl die E- als auch die Z-lsomeren, sowie die optischen lsomeren, beliebige Mischungen dieser Isomeren, sowie die möglichen tautomeren Formen beansprucht.

Das Verhältnis des Ketoxims der Formel (II) und des Ethylenoxids im ersten Reaktionsschritt des erfindungsgemäßen Verfahrens liegt zwischen 1:1 und 1:10, vorzugsweise zwischen 1:1 und 1:5, besonders bevorzugt zwischen 1:1 und 1:3, während die Base in einem Verhältnis von 0.05 bis 1 Moläquivalenten, bevorzugt von 0.05 bis 0.5 Moläquivalenten, besonders bevorzugt von 0.05 bis 0.3 Moläquivalenten, jeweils bezogen auf das Ketoxim, eingesetzt wird.

Bei der Säureumsetzung des erhaltenen 2-Hydroxyethylketoxims der Formel (III) zum Aminoglykol wird die verwendete Säure im molaren Verhältnis von 2:1 bis 10:1, vorzugsweise zwischen 2:1 und 5:1, besonders bevorzugt zwischen 1:1 und 3:1 eingesetzt, jeweils bezogen auf das 2-Hydroxyethylketoxim.

Die Umsetzung der Ketoxime der Formel (II) in Gegenwart von Ethylenoxid zu den 2-Hydroxyethylketoximen der Formel (III) erfolgt beispielsweise und bevorzugt bei 0 bis +50°C, besonders bevorzugt bei 0 bis +40°C und ganz besonders bevorzugt bei 0 bis +30°C.

Als besonders vorteilhaft kann herausgestellt werden, dass die Bildung der 2-Hydroxyethylketoxime auch bei Raumtemperatur unter hoher Selektivität abläuft.

Ein besonderer Vorteil des erfindungsgemäßen Verfahrens besteht auch darin, dass die verfahrensgemäß entstehenden 2-Hydroxyethylketoxime der allgemeinen Formel (III) in Wasser schlecht oder nicht löslich sind und einfach durch Phasentrennung aus der wässrigen Phase isoliert werden können.

Weiterhin ist als vorteilhaft zu erwähnen, dass alle Reaktionsschritte des erfindungsgemäßen Verfahrens, ohne zwischenzeitliche Aufreinigung/Isolation der Zwischenstufen/Zwischenprodukte, nacheinander durchgeführt werden können. Ebenso ist als vorteilhaft zu nennen, dass das durch die Verseifung des 2-Hydroxyethylketoximes der allgemeinen Formel (III) entstandene Aminoglykol quantitativ freigesetzt wird und das ebenfalls entstehende Keton schlecht oder nicht wasserlöslich ist, was die quantitative Rückführung des Ketons erlaubt. Durch Zugabe von beispielsweise Hydroxylamin zu dem Keton, wird das Ausgangsprodukt (Ketoxim) für einen erneuten Synthesezyklus wieder zur Verfügung gestellt. (siehe auch Schema (II))

Die erhaltenen 2-Hydroxyethylketoxime der Formel (III) sind aus der Literatur nicht bekannt und sind somit als neue Stoffe ebenfalls Gegenstand der vorliegenden Erfindung.

Als bevorzugte Verbindungen der allgemeinen Formel (III) sind zu nennen: 2-Hydroxyethylketoxime in denen (a) R1 einem Methyl und R einem Isobutyl entspricht, (b) R1 einem Methyl und R einem tert.-Butyl entspricht und (c) R1 einem

Ethyl und R einem tert.-Butyl entspricht.

Die Erfindung soll anhand der nachfolgenden Ausführungsbeispiele näher erläutert werden, ohne sie jedoch auf diese zu beschränken.

### Beispiel 1

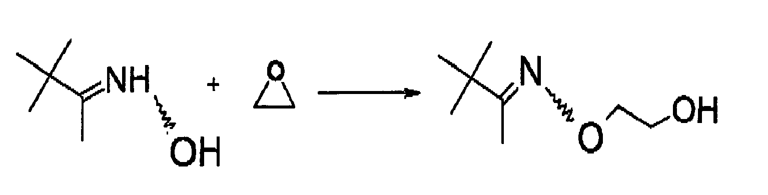

### 3,3-Dimethylbutan-2-one O-(2-hydroxyethyl)oxim

400 ml Wasser, 92 g (0.8 mol) 3,3-Dimethylbutan-2-one oxim und 3.4 g LiOH wurden vorgelegt und 70 g (1.6 mol) Ethylenoxid wurden so eingeleitet, dass das Gas vollständig aufgenommen wurde. Danach wurde das Gemisch für die Nachreaktion 8-12 Std. bei Raumtemperatur (RT) gerührt. Die obere Phase wurde abgetrennt, mit Ethylacetat versetzt und mit Wasser gewaschen. Die organische Phase wurde im Vakuum mit 100-200 mbar eingeengt. Rückstand: 114-120 g farbloses Öl, was einer Ausbeute von 90 % Ausbeute entspricht; Sdp. 106 °C/35 mbar.

NMR 1H (CDCI3): 1,11 (9H,s), 1,83 (3H,s), 3.2 (1H, bs), 3.85 (2H, m), 4.2 (2H,m) ppm.

### Beispiel 2

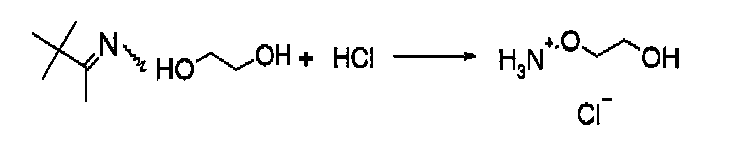

### (2-Hydroxyethoxy)ammonium chloride / Aminoglykolhydrochlorid

78.4 g Salzsäure (37.5 %, d 1.19) und 275 ml Wasser wurden vorgelegt und 44 g (0.275 mol) 3,3-Dimethylbutan-2-one O-(2-hydroxyethyl)oxlm wurden zugegeben. Das Gemisch wurde auf 100°C erhitzt und das Pinakolon/Wasser-Gemisch wurde binnen 3,5 h bei Normaldruck abdestilliert.

Der erhaltene Rückstand von 170 g wurde analysiert.

Titration: 18 % von Aminoglykolhydrochlorid; Die Ausbeute entspricht 95 - 97 % der Theorie.

### Beispiel 3

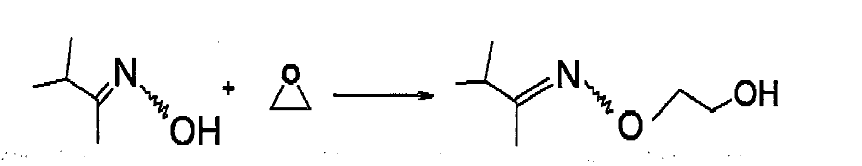

### 2-Methylbutan-2-one O-(2-hydroxyethyl)oxim

Die Reaktion wird analog zu der in Beispiel 1 beschrieben Reaktion durchgeführt, jedoch wird an Stelle des 3,3-Dimethylbutan-2-one-oxims 3-Methylbutan-2-one-oxim verwendet. Die Ausbeute entspricht 78% der Theorie.

## Patentansprüche

1. Verfahren zum Herstellen von Aminoglykol der Formel (I) 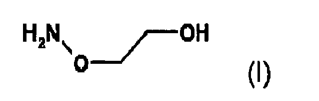 als Schritte enthaltend
(a) die Umsetzung eines Ketoxims der allgemeinen Formel (II) mit Ethylenoxid in wässriger Lösung und in Gegenwart einer Base 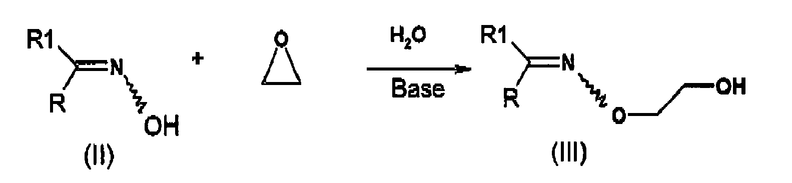 wobei
R1 und R unabhängig voneinander unsubstituiertes oder durch Halogen, CN, NO₂, einfach oder mehrfach substituiertes lineares (C₁-C₈)-Alkyl, Phenyl oder verzweigtes (C₃-C₈)-Alkyl bedeuten, aber für den Fall, dass R1 oder R ein unsubstituiertes oder durch Halogen, CN, NO₂ substituiertes lineares (C₁-C₈)-Alkyl ist, entspricht der jeweils andere Rest R1 oder R einem unsubstituierten oder durch Halogen, CN, NO₂ substituierten Phenyl oder einem vezweigten (C₃-C₈)-Alkyl.
und (b) 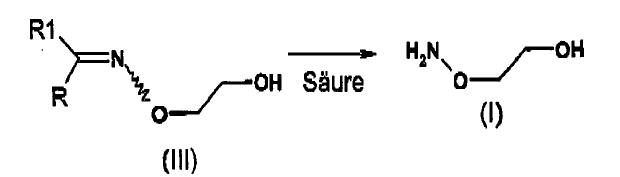 die Weiterreaktion des gemäß des Reaktionsschritts (a) entstandenen 2-Hydroxyethylketoxims der allgemeinen Formel (III) in Gegenwart einer Säure zum Aminoglykol.

2. Verfahren gemäß Anspruch 1, **dadurch gekennzeichnet, dass** R1 und R die Bedeutung von Methyl, Ethyl, Propyl, Phenyl, sek.-Butyl, tert.-Butyl, Isobutyl, Isopropyl oder Neopentyl haben, wobei die vorgenannten Reste jeweils unabhängig voneinander unsubstituiert oder durch Halogen, CN, NO₂, einfach oder mehrfach substituiert sind, aber für den Fall, dass R1 oder R ein unsubstituiertes oder durch Halogen, CN, NO₂ substituiertes Methyl, Ethyl oder Propyl ist, entspricht der jeweils andere Rest R1 oder R einem unsubstituierten oder durch Halogen, CN, NO₂ substituierten Phenyl, sek.-Butyl, tert.-Butyl, Isobutyl, Isopropyl oder Neopentyl.

3. Verfahren gemäß Anspruch 1, **dadurch gekennzeichnet, dass** R1 und R die Bedeutung von Methyl, Ethyl, Phenyl, sek.-Butyl, tert.-Butyl, Isobutyl oder Isopropyl haben, wobei für den Fall, dass R1 oder R ein Methyl oder Ethyl ist, der jeweils andere Rest ausgewählt sein muss aus der Gruppe Phenyl, sek.-Butyl, tert.-Butyl, Isobutyl oder Isopropyl.

4. Verfahren gemäß Anspruch 1, **dadurch gekennzeichnet, dass** R1 einem Methyl und R einem tert-Butyl, oder Isobutyl entspricht, oder aber R einem Methyl und R1 einem tert-Butyl, oder Isobutyl entspricht.

5. Verfahren gemäß Anspruch 1, **dadurch gekennzeichnet, dass** R1 einem Methyl und R einem tert.-Butyl entspricht, oder aber R einem Methyl und R1 einem tert.-Butyl entspricht.

6. Verfahren gemäß einem der Ansprüche 1 bis 5, **dadurch gekennzeichnet, dass** als Base in Reaktionsschritt (a) eine organische oder anorganische Basen verwendet wird.

7. Verfahren gemäß einem der Ansprüche 1 bis 5, **dadurch gekennzeichnet, dass** als Base in Reaktionsschritt (a) NaOH, LiOH oder KOH verwendet wird.

8. Verfahren gemäß einem der Ansprüche 1 bis 7, **dadurch gekennzeichnet, dass** als Säure in Reaktionsschritt (b) eine organische oder anorganische Säure verwendet wird.

9. Verfahren gemäß einem der Ansprüche 1 bis 7, **dadurch gekennzeichnet, dass** als Säure in Reaktionsschritt (b) HCI, HBr, HF, H₂S0₄, H₃PO₄, H₃BO₃, CH₃COOH, CF₃COOH oder p-Toluolsulfonsäure verwendet wird.

10. Verfahren gemäß einem der Ansprüche 1 bis 7 **dadurch gekennzeichnet, dass** als Säure HCI oder H₂SO₄ verwendet wird.

11. Verfahren gemäß einem der Ansprüche 1 bis 10, **dadurch gekennzeichnet, dass** das Verhältnis des Ketoxims der Formel (II) und des Ethylenoxids im ersten Reaktionsschritt (a) zwischen 1:1 und 1:10 liegt.

12. Verfahren gemäß einem der Ansprüche 1 bis 11, **dadurch gekennzeichnet, dass** die im zweiten Reaktionsschritt (b) verwendete Säure im molaren Verhältnis von 2:1 bis 10:1, bezogen auf das 2-Hydroxyethylketoxim eingesetzt wird.

13. Verfahren gemäß einem der Ansprüche 1 bis 12, **dadurch gekennzeichnet, dass** die Reaktionsschritte in einem Temperaturbereich von 0°C bis +50°C erfolgen.

14. 2-Hydroxyethylketoxime der allgemeinen Formel (III), 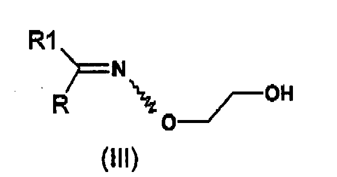 wobei
R1 und R unabhängig voneinander unsubstituiertes oder durch Halogen, CN, NO₂, einfach oder mehrfach substituiertes lineares (C₁-C₈)-Alkyl, Phenyl oder verzweigtes (C₃-C₈)-Alkyl bedeuten, aber für den Fall, dass R1 oder R ein unsubstituiertes oder durch Halogen, CN, N0₂ substituiertes lineares (C₁-C₈)-Alkyl ist, entspricht der jeweils andere Rest R1 oder R einem unsubstituierten oder durch Halogen, CN, NO₂ substituierten Phenyl oder einem vezweigten (C₃-C₈)-Alkyl.

15. 2-Hydroxyethylketoxim gemäß Anspruch 14, **dadurch gekennzeichnet ist, dass** R1 einem Methyl und R einem Isobutyl entspricht.

16. 2-Hydroxyethylketoxim gemäß Anspruch 14, **dadurch gekennzeichnet ist, dass** R1 einem Methyl und R einem tert.-Butyl entspricht.

17. 2-Hydroxyethylketoxim gemäß Anspruch 14, **dadurch gekennzeichnet ist, dass** R1 einem Ethyl und R einem tert.-Butyl entspricht.

18. Verwendung der 2-Hydroxyethylketoxime gemäß einem der Ansprüche 14 bis 17 zur Herstellung von Aminoglykol.
